# FASCICULE DE BREVET EUROPEEN

(11) **EP 2 417 251 B1**
(45) Date de publication et mention de la délivrance du brevet: **02.07.2014**
(21) Numéro de dépôt: 10723229.0
(22) Date de dépôt: 08.04.2010
(51) Int. Cl.: C12N 7/02, A61K 39/205

(54) **PROCEDE DE PURIFICATION DU VIRUS RABIQUE**
VERFAHREN ZUR AUFREINIGUNG DES TOLLWUTVIRUS
METHOD FOR PURIFYING THE RABIES VIRUS

(30) Priorité: 08.04.2009 FR 0952310; 05.08.2009 US 231394 P
(43) Date de publication de la demande: 15.02.2012
(60) Demande divisionnaire: 14170027.8
(73) Titulaire: Sanofi Pasteur, 69367 Lyon Cedex 07 (FR)
(72) Inventeur: FABRE, Virginie, F-69006 Lyon (FR); ROCCA, Céline, F-69690 Bibost (FR); RIFFARD, Pierre, F-69380 Dommartin (FR); CALVOSA, Eric, F-69610 Haute Rivoire (FR)
(86) Numéro de dépôt international: PCT/FR2010/050679
(87) Numéro de publication internationale: WO 2010/116096

(56) Documents cités:
- EP-A- 0 171 771
- EP-A- 1 724 338
- WO-A-97/06243
- WO-A-2008/071774
- FRAZATTI-GALLINA N M ET AL: "Vero-cell rabies vaccine produced using serum-free medium" VACCINE, BUTTERWORTH SCIENTIFIC. GUILDFORD, GB, vol. 23, no. 4, 9 décembre 2004 (2004-12-09), pages 511-517, XP004629187 ISSN: 0264-410X cité dans la demande
- JUNGBAUER ET AL: "Polymethacrylate monoliths for preparative and industrial separation of biomolecular assemblies" JOURNAL OF CHROMATOGRAPHY, ELSEVIER SCIENCE PUBLISHERS B.V. AMSTERDAM, NL, vol. 1184, no. 1-2, 9 janvier 2008 (2008-01-09), pages 62-79, XP022503329 ISSN: 0021-9673

## Description

L'invention a pour objet une méthode de purification du virus rabique qui a été obtenu à partir d'une culture de cellules.

D'une façon générale, les récoltes de virus obtenues à partir de cultures cellulaires infectées contiennent non seulement les virus désirés mais aussi les protéines et l'ADN des cellules qui sont des impuretés dont il convient de se débarrasser. Les quantités en protéines et en ADN cellulaires relarguées sont d'autant plus importantes que les virus sont responsables d'une lyse cellulaire importante et/ou que les récoltes virales sont effectuées tardivement. Outre les impuretés d'ordre cellulaire, les protéines du milieu dans lequel se trouvent les cellules infectées sont également des impuretés dont on cherche aussi à se débarrasser lors de la mis en oeuvre du procédé de purification virale.

Lorsque les virus servent à la fabrication de vaccins on cherche à obtenir des préparations qui soient les plus pures possible pour éviter le développement de réactions allergiques contre les impuretés protéiques. Dans certains pays, il existe une législation limitant à 100 pg/dose vaccinale voire moins la quantité maximale autorisée d'ADN cellulaire dans les vaccins comprenant des produits obtenus à partir de lignées cellulaires continues.

Différents procédés de purification du virus rabique ont déjà été décrits dans l'art antérieur :
U.S. 4,664,912 décrit une méthode de purification du virus rabique par centrifugation zonale après qu'il ait été inactivé à la β propiolactone. Une autre méthode consiste à combiner une chromatographie par exclusion de taille et une chromatographie par échange d'anions lorsque le volume de la récolte de virus à purifier est important.
U.S. 4,725,547 décrit une méthode de purification du virus rabique par chromatographie d'affinité sur cellulofine sulfate (ester d'acide sulfurique et de cellulose).
WO 97/06243 décrit une méthode de purification des virus, notamment du virus rabique à partir d'une culture de cellules Vero infectées. Le procédé comprend une chromatographie par échange d'anions suivie d'une chromatographie par échangeuse de cations et est complété par une chromatographie d'affinité par chélation métallique. En utilisant ce procédé, le taux d'ADN résiduel contenu dans une dose de vaccin est ≤ à 30-40 pg en utilisant la technique dite du « Threshold Total DNA assay ».
Kumar A.P et coll dans Microbes and Infection (2005) 7 ; 1110-1116 a comparé deux procédés de purification du virus rabique à partir d'un surnageant de culture de cellules Vero infectées qui ont été cultivées dans un milieu contenant du sérum de veau foetal. Il montre que le procédé de purification basé sur l'utilisation d'une colonne de chromatographie échangeuse d'anions (DEAE-sepharose CL -6B) est plus performant que le procédé de purification par centrifugation zonale sur gradient de saccharose puisqu'à degré de pureté comparable en terme de quantité d'acides nucléiques et de protéines résiduelles, le rendement en virus rabique est meilleur avec le procédé chromatographique.
Frazatti-Gallina N.M. dans Vaccine (2004) 23 ; 511-517 a décrit également un procédé de purification du virus rabique à partir d'un surnageant de culture de cellules Vero infectées qui ont été cultivées dans un milieu dépourvu de sérum de veau foetal. Le procédé de purification met en oeuvre également une étape de chromatographie par échange d'anions sur un support à base de DEAE-cellulose après une étape de clarification et de concentration de la récolte virale. En utilisant ce procédé, la quantité d'ADN résiduel mesurée par la technique d'hybridation sur blot est <23 pg par dose vaccinale.

Ainsi, lorsque le procédé de purification du virus rabique comprend une étape de chromatographie par échange d'ions, on retrouve quasi systématiquement une étape de chromatographie par échange d'anions de façon à retenir les acides nucléiques du milieu contenant le virus à purifier sur le support chromatographique.

D'autre part, JungBauer et coll. ont décrit dans Journal of chromatography (2008), vol 184, pp 62-79 les potentialités d'un nouveau support chromatographique monolithique pour purifier avec un très bon degré de pureté le virus de la mosaïque du tabac (VMT).

Un procédé de purification de virus particulièrement performant devrait permettre d'éliminer de façon optimale les impuretés protéiques et l'ADN cellulaire tout en garantissant un rendement maximal en virus rabique purifié et il existe toujours le besoin de trouver de nouveaux procédés qui répondent à ces critères.

Un but de l'invention est de fournir un nouveau procédé de purification qui répond à ces critères.

Un autre but de l'invention est de fournir un procédé pour purifier du virus rabique à partir d'une récolte de virus dépourvue de sérum animal ou de toute protéine sérique et plus particulièrement de fournir un procédé de purification où on utilise uniquement des produits d'origine non animale.

A cet effet, la présente invention a pour objet :
Un procédé de purification du virus rabique comprenant une seule étape de chromatographie par échange d'ions, la dite étape étant une chromatographie par échange de cations selon laquelle :
   a. on met en contact le surnageant d'une culture de cellules infectées par ce virus avec un support de chromatographie échangeur de cations comprenant une matrice en polyméthacrylate sur laquelle ont été greffés par liaison de covalence des groupements sulfoisobutyl de sorte que le virus rabique se fixe sur ce support, et dans un deuxième temps, et
   b. on élue le virus de son support.

Selon un aspect du procédé selon l'invention, le surnageant de culture de cellules infectées par le virus rabique est dépourvu de sérum animal ou de toute protéine sérique.

Selon un autre aspect, le surnageant de culture de cellules infectées par le virus rabique est dépourvu de toute protéine exogène d'origine animale.

Selon encore un autre aspect, la concentration en protéines exogènes d'origine non animale dans le surnageant de culture de cellules infectées par le virus rabique est ≤ 15 mg/l.

Selon encore un autre aspect, le surnageant de culture de cellules infectées par le virus rabique est dépourvu de tout produit exogène d'origine animale.

Selon un mode de réalisation du procédé selon l'invention, le surnageant de culture de cellules infectées par le virus rabique est préalablement clarifié avant d'être mis en contact avec le support de chromatographie échangeur de cations.

Le procédé de purification selon l'invention est caractérisé en ce que la quantité de virus mesurée dans l'éluât correspond à au moins 70% de la quantité de virus mesurée dans le surnageant qui a été mis en contact avec le support chromatographique.

Plus particulièrement, il est caractérisé en ce que la quantité de protéines totales mesurée dans l'éluât correspond à moins de 40% de la quantité de protéines totales mesurée dans le surnageant qui a été mis en contact avec le support chromatographique et en ce que la quantité d'ADN mesurée dans l'éluât correspond à moins de 5%, de préférence à moins de 2,5% et de façon encore plus préférée à moins de 1% de la quantité d'ADN mesurée dans le surnageant qui a été mis en contact avec le support chromatographique.

Selon un autre mode de réalisation du procédé selon l'invention, après avoir élué le virus de son support chromatographique, on le concentre éventuellement puis on le traite avec une nucléase.

Selon un aspect du procédé selon l'invention, la nucléase est une endonuclease, telle que la benzonase.

Dans un autre mode de réalisation de l'invention, on soumet ensuite l'éluât à une ultra centrifugation sur un gradient de saccharose et on recueille la ou les fractions du gradient qui contiennent le virus purifié.

Dans un autre aspect de l'invention, on inactive ensuite le virus rabique purifié au moyen d'un agent d'inactivation virale.

Dans un aspect particulier, l'agent d'inactivation virale est la β-propiolactone.

Selon un aspect préféré toutes les étapes du procédé selon l'invention sont réalisées au moyen de produits d'origine non animale.

L'invention a également pour objet un procédé de fabrication d'un vaccin contre la rage selon lequel :
a) on infecte une culture de cellules avec le virus rabique,
b) on purifie le virus rabique à partir du surnageant de culture de cellules infectées selon un procédé de l'invention,
c) on mélange la suspension de virus purifié obtenue en b) dans un tampon de conservation, et
d) on répartit la suspension de virus purifié obtenue en c) sous la forme de vaccins uni-doses ou multi-doses.

Selon un autre aspect, il s'agit d'un procédé de fabrication d'un vaccin contre la rage selon lequel :
a) on infecte une culture de cellules avec le virus rabique,
b) on purifie le virus rabique à partir du surnageant de culture de cellules infectées selon un procédé de l'invention,
c) on mélange la suspension de virus purifié obtenue en b) dans un tampon de lyophilisation,
d) on répartit le mélange obtenu en c) sous la forme de vaccins uni-doses ou multidoses, et
e) on lyophilise les doses de vaccins.

### Description détaillée de l'invention

Le procédé de purification du virus rabique selon l'invention comprend une seule étape de chromatographie par échange d'ions, la dite étape chromatographique étant une chromatographie par échange de cations. Il est bien entendu qu'au sens de la présente invention le procédé de purification du virus rabique ne se limite pas une seule étape de chromatographie mais peut inclure une ou plusieurs autres étapes supplémentaires dans la mesure où ces étapes ne sont pas des chromatographies par échange d'ions. Contrairement à ce que laisse envisager l'état de l'art, les inventeurs ont mis en évidence que les performances d'une chromatographie par échange de cations dont le support comprend une matrice de polymethacrylate sur laquelle ont été greffés par liaison de covalence des groupements sulfoisobutyl sont meilleures que celles d'une chromatographie par échange d'anions. Sans vouloir être lié par la théorie, il semble que le virus rabique possède une double polarité, positive et négative qui lui permet de se fixer à la fois sur des supports échangeurs d'anions (type DEAE) et sur des supports échangeurs de cations (type SO₃⁻). Les « performances d'une chromatographie » au sens de l'invention sont calculées sur la base de la quantité de virus rabique et d'ADN que l'on retrouve dans l'éluât par rapport à la quantité initiale de virus et d'ADN qui ont été mis en contact avec le support chromatographique, dans les limites de capacité de charge du support chromatographique. A quantités équivalentes de virus et d'ADN qui ont été mis en contact avec le support chromatographique, la performance du support chromatographique est d'autant meilleure que la quantité de virus retrouvé dans l'éluât est importante et que la quantité d'ADN résiduel est faible. Les groupements sulfoisobutyl ont été greffés à la matrice de polymethacrylate au moyen de chaînes polymériques flexibles qui facilitent l'interaction du virus rabique avec l'échangeur de cations. De façon préférée, la chaîne polymérique comprend un enchainement d'unités monomériques ayant pour formule :

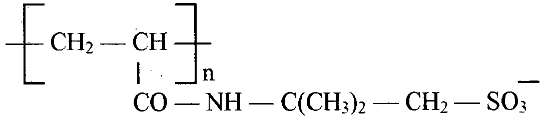

Ces chaînes polymériques sont obtenues par polymérisation d'un monomère ayant pour formule chimique : CH₂=CH-CO-NH-C(CH₃)₂-CH₂-SO₃- en présence d'un catalyseur à base de cérium qui permet également le greffage à la matrice de polyméthacrylate. Elles comprennent en moyenne entre 15 et 25 unités monomériques. Le support chromatographique est classiquement sous la forme de particules dont la taille varie généralement entre 20 et 100 µm, de façon préférée entre 40 et 90 µm. Ce type de support est notamment commercialisé par MERCK sous la dénomination de Fractogel ® EMD SO3⁻. Ce type de support est placé dans une colonne chromatographique dont la longueur et le diamètre sont choisis en fonction du volume de récolte à purifier. Les résultats présentés dans l'exemple 1 montrent qu'on obtient de bonnes performances uniquement avec le support Fractogel ® EMD S03- dans l'ensemble des supports chromatographiques échangeurs d'anions et de cations qui ont été testés. On obtient des rendements en virus très faibles (moins de 10% du virus introduit est récupéré dans L'éluât) avec tous les autres supports chromatographiques échangeurs de cations forts testés. Les rendements en virus obtenus avec les supports chromatographiques échangeurs d'anions sont meilleurs (entre 40 et 70%) mais restent néanmoins inférieurs au rendement en virus que l'on obtient avec le support Fractogel ® EMD S03- qui est en général supérieur à 70 % et le plus souvent supérieur à 80%. Par ailleurs on élimine moins d'ADN avec les supports chromatographiques échangeurs d'anions. De façon surprenante, seul le support chromatographique échangeur de cations comprenant une matrice de polyméthacrylate sur laquelle ont été greffés par liaison de covalence des groupements sulfoisobutyl qui jouent le rôle de ligands permet de purifier avec de bonnes performances le virus à partir d'un surnageant de culture de cellules infectées par le virus rabique. On élimine en une étape chromatographique à la fois plus de 60% des protéines totales, au moins 2,5 log₁₀ d'ADN, de façon préférée au moins 3,0 log₁₀ d'ADN et de façon encore plus préférée au moins 3,5 log₁₀ d'ADN (ce qui correspond à une élimination de plus de 99% de l'ADN cellulaire) tout en conservant un rendement en virus rabique d'au moins 70%, (c'est à dire que l'on retrouve dans l'éluât une quantité de virus rabique qui correspond au moins à 70% de celle qui a été initialement mise en contact avec le support chromatographique) et de façon préférée d'au moins 80%. Ces performances sont observées notamment lorsque le surnageant de culture de cellules infectées par le virus rabique ne contient pas de sérum animal ou de protéine sérique comme l'albumine. Ainsi les quantités de protéines totales et d'ADN mesurées dans l'éluât chromatographique contiennent respectivement moins de 40 % de la quantité de protéines totales présente dans le volume de surnageant de culture de cellules infectées par le virus rabique qui a été mis en contact avec le support chromatographique et moins de 5%, de préférence moins de 2,5 % et de façon encore plus préférée moins de 1% de la quantité d'ADN également présente dans le volume de surnageant de culture de cellules infectées par le virus rabique qui a été mis en contact avec le support chromatographique. Par contre, au moins 70% et de façon préférée au moins 80% de la quantité de virus rabique présente dans le volume de surnageant qui a été mis en contact avec le support chromatographique est retrouvée dans l'éluât.

Les « protéines totales » au sens de l'invention représentent toutes les protéines qui sont présentes dans le matériel analysé. Cela comprend les protéines du virus rabique, les protéines cellulaires, les protéines du milieu de culture cellulaire et/ou du milieu d'infection virale et les protéines qui sont introduites lors de la mise en oeuvre du procédé de procédé de production et de purification du virus rabique (par exemple la trypsine et/ou la benzonase). Les protéines cellulaires et les protéines du milieu de culture et/ou du milieu d'infection virale de même que les protéines qui ont été introduites durant la production et/ou la purification du virus rabique représentent les impuretés (impuretés protéiques) que l'on cherche à éliminer tandis que l'on cherche à conserver au maximum les protéines du virus rabique. Les protéines du virus rabique sont la glycoprotéine G, la nucléoprotéine N, la phosphoprotéine P, la protéine de matrice M, la RNA polymerase L.

La quantité de virus rabique est évaluée sur la base de la mesure de la glycoprotéine G du virus rabique. Elle se fait habituellement par une méthode ELISA de type « sandwich » en utilisant deux anticorps qui reconnaissent de préférence deux épitopes conformationnels de la protéine G, comme cela est décrit dans l'exemple 1. Par exemple on peut utiliser un anticorps neutralisant reconnaissant un épitope conformationnel localisé sur le site antigénique IIc de la glycoprotéine G (Journal of Clinical investigation (1989), vol. 84, pp 971 à 975) comme anticorps de capture et un anticorps neutralisant reconnaissant un épitope conformationnel localisé sur le site antigénique III de la glycoprotéine G (Biologicals (2003), vol. 31, pp 9 à 16) comme anticorps de révélation. Les résultats sont exprimés en UI sur la base de l'utilisation d'un étalon de référence, qui a été calibré vis-à-vis de la référence internationale du NIBSC.

Pour mesurer la quantité de protéines totales on utilise la méthode de Bradford bien connue de l'homme du métier.

Pour mesurer la quantité d'ADN, on utilise de préférence une méthode de PCR quantitative (qPCR) basée sur la quantification d'un fragment d'ADN du génome de la cellule (de préférence on cible un fragment d'ADN qui est répété de nombreuses fois dans le génome de la cellule). Lorsque le virus rabique est produit à partir de cellules Vero, la quantification de l'ADN résiduel durant le procédé de purification du virus est basée sur la quantification du fragment d'ADN alpha satellite du singe vert africain après amplification PCR en utilisant une méthode similaire à celle qui est décrite par Lebron J.A. et coll. dans Developments in Biologicals (2006), vol 123, pp35-44 et qui est détaillée dans l'exemple 1. Cette méthode est très avantageuse car elle permet de mesurer tous les ADN cellulaires qui ont plus de 200 paires de bases.

Le surnageant de culture contenant le virus rabique est produit à partir d'une culture de cellules qui ont été infectées par le virus rabique. Toute culture de cellules dans lesquelles le virus rabique se réplique convient à l'objet de l'invention. Il peut s'agir de cultures primaires de tissus animaux, comme les cultures primaires embryonnaires de poulets (par exemple les cultures primaires de fibroblastes embryonnaires de poulets (PECF)), les cultures primaires de cerveaux de souriceaux, les cultures primaires de reins de singe, de lapin, de hamster ou de chien, mais de façon préférée, on utilise une culture de cellules provenant de lignées cellulaires établies qui dérivent des cultures primaires de tissus animaux. Il s'agit notamment des lignées de cellules provenant de primates telles que la lignée VERO, WI-38, MRC5, PERC.6, la lignée 293, de chevaux, de vaches (telle que la lignée MDBK), de moutons, de chiens (telles que la lignée MDCK), de chats, de rongeurs (telles que la lignée BHK21, HKCC ou CHO).

De façon particulièrement préférée, on utilise la lignée de cellules Vero qui comporte de nombreux avantages: c'est une lignée continue facilement cultivable à l'échelle industrielle qui a un très faible pouvoir mutagène et qui est très sensible au virus rabique.

Les cellules peuvent être cultivées en suspension ou sur un support selon qu'elles ont des propriétés d'adhésion ou non, en mode batch, feed batch ou selon un mode de culture en continu et par perfusion. Dans le cas de culture de lignées cellulaires à l'échelle industrielle ou semi-industrielle, on utilise généralement des biogénérateurs, dont le volume est supérieur à 10 litres et pouvant aller jusqu'à plus de 2000 litres, comprenant un système d'agitation, un dispositif d'injection d'un courant gazeux de CO₂ et un dispositif d'oxygénation. Ils sont équipés de sondes mesurant les paramètres internes du bio-générateur comme le pH, l'oxygène dissous, la température, la pression de la cuve ou certains paramètres physicochimiques de la culture (comme la consommation en glucose, de glutamine ou la production de lactates et d'ions ammoniums). Les sondes de pH, d'oxygène et de température sont reliées à un bio-processeur qui assure en permanence la régulation de ces paramètres. Dans le cas de lignées cellulaires adhérentes cultivées en biogénérateurs, le milieu de culture contient des microporteurs qui sont des microbilles sur lesquelles se fixent les cellules. Ces microporteurs sont maintenus en suspension par agitation mécanique, ou au moyen d'un courant gazeux. Dans le cas des cellules de la lignée Vero ont utilise habituellement des microporteurs dont la matrice électrostatique adhésive est à base de dextran substituée par des groupements N, N diethylaminoéthyle qui sont commercialisés notamment par Amersham Biosciences sous la dénomination de Cytodex 1, ou Cytodex 2. On peut également utiliser comme microporteurs des microbilles de Cytodex 3 commercialisés par Amersham Biosciences.

Le milieu qui sert à la culture des cellules, encore appelé milieu de culture cellulaire peut être supplémenté ou non en sérum d'origine animale, ou contenir une ou plusieurs protéines sériques comme l'albumine, ou être dépourvu de toute protéine d'origine animale, ou même être dépourvu de toute protéine. De façon préférée, on utilise un milieu de culture cellulaire dépourvu de tout sérum animal ou de toute protéine sérique, telle que l'albumine qui peut être à l'origine du développement de réaction d'hypersensibilité chez le sujet vacciné (Swanson MC et coll, Journal of Infectious Disease (1987) ; 155(5) 909-913). De façon particulièrement préférée, on utilise un milieu de culture dépourvu de toute protéine d'origine animale, ou même de tout produit d'origine animale. Par « protéine ou produit d'origine animale », on entend une protéine ou un produit dont le procédé de fabrication comprend au moins une étape où on utilise une matière provenant de l'animal ou de l'homme. Cela permet de diminuer les risques de transmission de maladies telles que la BSE qui peuvent être liés à l'utilisation des produits biologiques d'origine animale. Le milieu de culture contient généralement de très faibles quantités de protéines produites par exemple sous forme de protéines recombinantes ou extraites de végétaux (soja, riz...) ou de levures. La concentration en protéines est généralement ≤ 15 mg/l mesurée par la méthode de Bradford. Souvent, il contient essentiellement des protéines de bas poids moléculaire (≤ 10 KDA) qui sont en fait des polypeptides. C'est le cas en particulier du milieu VP SFM commercialisé par Invitrogen qui convient pour le procédé selon l'invention, notamment pour la culture des cellules Vero On cite également les milieux Opti Pro ^{™} serum-free (InVitrogen), Episerf (InVitrogen), Ex-cell ® MDCK (Sigma-Aldrich), Ex-Cell ^{™} Vero (SAFC biosciences) MP-BHK® serum free (MP Biomedicals), SFC-10 BHK express serum free (Promo cell), SFC-20 BHK express protein free (Promo cell), HyQ PF Vero (Hyclone Réf. SH30352.02), Hyclone SFM4 Megavir , le milieu MDSS2 (Axcell biotechnology), le milieu DMEM Iscove modifié (Hyclone), les milieux nutritifs de Ham (Ham -F10, Ham-F12), le milieu de leibovitz L-15 (Hyclone) qui sont des milieux dépourvus de tout produit d'origine animale et qui contiennent généralement de faibles quantités de protéines (≤ 15 mg/l).

Le virus rabique peut provenir de n'importe quelle origine pourvu qu'il se reproduise dans les cellules sensibles au virus rabique. On utilise généralement des souches de virus rabiques qui ont été établies à partir d'isolats primaires, comme la souche Pasteur 2061, la souche VP-11, la souche Pitman-Moore 1.503-3 M. Ces souches très virulentes sont destinées à la fabrication de vaccins inactivés contre la rage. On peut également utiliser des souches de virus rabiques atténuées dans le but de produire un vaccin vivant atténué contre la rage. Il s'agit par exemple de la souche SAD Bern ou de la souche SAD B19 ou des souches dérivées de celles-ci, comme les souches SAG1 et SAG2 qui dérivent de la souche SAD Bern du fait de l'existence de mutations ponctuelles au niveau de la glycoprotéine G du virus rabique (EP350398, EP583998). EP1253197 décrit également d'autres souches de virus rabiques atténués plus stables qui comportent une ou plusieurs mutations ponctuelles au niveau de la phosphoprotéine P et de la glycoprotéine G du virus rabique.

Le surnageant de culture des cellules infectées par le virus rabique est obtenu en utilisant un milieu d'infection virale qui remplace le milieu de culture cellulaire au moment de l'infection des cellules et qui sert à la production du virus rabique par les cellules infectées. La composition chimique du milieu d'infection virale peut être identique ou être différente de la composition du milieu de culture cellulaire. Le milieu d'infection virale comme le milieu de culture cellulaire peut être supplémenté en sérum d'origine animale ou en une ou plusieurs protéines sériques, mais de façon préférée, le milieu d'infection virale est dépourvu de tout sérum animal ou de toute protéine sérique pour les raisons invoquées précédemment. De façon particulièrement préférée, le milieu d'infection virale est dépourvu de toute protéine d'origine animale, ou même de tout produit d'origine animale pour prévenir les risques de transmission de maladies telles que la BSE qui peuvent être liés à l'utilisation des produits biologiques d'origine animale. Il contient généralement de très faibles quantités de protéines produites par exemple sous forme de protéines recombinantes ou extraites de végétaux (soja, riz...) ou de levures. La concentration en protéines est généralement ≤ 15 mg/l mesurée par la méthode de bradford. Souvent, il contient essentiellement des protéines de bas poids moléculaire (≤ 10 kDa) qui sont en fait des polypeptides. Dans un aspect particulier, le milieu d'infection virale ne contient pas de protéine ayant un poids moléculaire supérieur à 10 kDa. Comme milieu d'infection virale dépourvu de tout produit d'origine animale et faiblement dosé en protéines (concentration ≤ 15 mg/l), on cite le milieu VP SFM (Invitrogen), le milieu de leibovitz L-15, le milieu MEM (Sigma-Aldrich), le milieu Hyclone SFM4Megavir ^{™} (Hyclone) ou éventuellement un des milieux à base d'extraits végétaux tels que décrits dans la demande WO 99/4768. Par exemple, lorsque le virus rabique est produit à partir d'une culture de cellules de la lignée Vero, le surnageant de culture des cellules infectées est généralement obtenu en utilisant comme milieu d'infection virale un milieu dépourvu de toute protéine sérique ou même de tout produit d'origine animale comme ceux cités précédemment.

En fonction de la composition du milieu de culture cellulaire et du milieu d'infection virale, le surnageant de culture des cellules infectées par le virus rabique peut avantageusement :
- être dépourvu de toute protéine sérique ou,
- être dépourvu de toute protéine exogène d'origine animale, ou même
- être dépourvu de tout produit exogène d'origine animale.
Lorsque le surnageant de culture de cellules infectées par le virus rabique est dépourvu de toute protéine exogène ou est dépourvu de tout produit exogène d'origine animale, les protéines exogènes d'origine non animale que l'on retrouve dans le surnageant sont en général à une concentration très faible (≤ 15 mg/l) mesurée par la méthode de bradford.

Par « protéine ou produit exogène », on entend tout produit ou toute protéine qui n'est pas un composant du virus rabique ou des cellules qui servent à la production du virus rabique. Les protéines ou les produits exogènes sont des composants qui sont introduits lors de la production et/ou de la purification du virus rabique. Par exemple, les protéines ou les produits qui sont présentes dans la composition des milieux de culture, les enzymes tels que la trypsine ou la benzonase qui sont introduites ou les produits qui sont introduits lors de la production et/ou la purification du virus rabique sont des protéines ou des produits exogènes. Les protéines/produits exogènes sont d'origine animale lorsque leur procédé de fabrication comprend au moins une étape où on utilise une matière provenant de l'animal ou de l'homme. Les protéines/produits exogènes sont d'origine non animale lorsqu'ils sont fabriqués par d'autres moyens, par exemple en utilisant une matière végétale, par synthèse chimique ou par recombinaison génétique en utilisant des levures, des bactéries ou des plantes.

La récolte des surnageants de culture contenant le virus infectieux débute en général 2 à 3 jours après avoir infecté les cellules, et se poursuit pendant une quinzaine jours car le virus est peu lytique. Après chaque récolte, on remet en culture les cellules infectées avec du milieu d'infection virale neuf. Les protéines que l'on retrouve dans les surnageants de culture ont une origine cellulaire, virale ou proviennent du milieu d'infection virale et dans une très faible mesure du milieu de culture cellulaire. On peut conserver toutes les récoltes ou garder uniquement les récoltes qui ont des titres infectieux élevés. Les récoltes peuvent être conservées individuellement. On peut également mélanger plusieurs récoltes. On garde habituellement les récoltes à une température voisine de +5°C ou sous forme congelée.

Par précaution, on clarifie en général le surnageant de culture contenant le virus à purifier avant l'étape de chromatographie par échange de cations de façon à éliminer les débris cellulaires grossiers, les agrégats éventuellement présents, et les microporteurs résiduels lorsque la culture cellulaire a été réalisée sur microporteurs. Toute méthode de clarification bien connue de l'homme de l'art peut être appliquée dans le procédé selon l'invention. On peut par exemple centrifuger les surnageants ou procéder à une chromatographie par exclusion de taille pour clarifier le surnageant de culture. Habituellement, la clarification est réalisée par filtration tangentielle, par filtration frontale sur membrane et/ou par filtration en profondeur en utilisant un ou plusieurs filtres dont la porosité varie habituellement entre 0,2 et 1,5 µm, de préférence entre 0,4 et 1,0 µm. La clarification peut être réalisée au moyen d'une seule filtration du surnageant de culture dans les cas où il y a peu de débris et d'agrégats. La clarification peut être réalisée aussi en combinant au moins deux filtrations successives, la première filtration se faisant par exemple avec un filtre relativement grossier ayant par exemple une porosité comprise entre 0,3 et 1,5 µm tandis que la deuxième filtration se fait avec un filtre relativement fin ayant par exemple une porosité comprise entre 0,2 et 0,5 µm. Au préalable on peut également prévoir une étape de pré-filtration en utilisant un pré-filtre dont la porosité est importante (entre 2 et 10 µm) pour éliminer les gros débris. Comme filtres convenant à l'objet de l'invention on cite les filtres en cellulose, les fibres de cellulose régénérés, les fibres de cellulose combinés à des filtres inorganiques (par exemple à base de diatomées, de perlite, ou de silice fumée), les filtres de cellulose combinés à des filtres inorganiques ou des résines organiques, les filtres à base de polymères, tels que par exemple les filtres en nylon, en polypropylène, en polyvinylidène fluoré ou en polyethersulfone. Ces filtres sont notamment commercialisés sous le nom de Durapore®, de Millipak®, de Millidisk^{™} distribué par la société Millipore ou les filtres distribués par la société Pall. Comme système de filtres en profondeur, on cite les filtres en profondeur de la série AP (AP01, de la série CP (CP10, CP30, CP50, CP60, CP70, CP90), de la série HP (HP10, HP30, HP50, HP60, HP70, HP90), de la série CA (CA10, CA30, CA50, CA60, CA70, CA90) et de la série SP (SP10, SP30, SP50, SP60, SP70, SP90) qui sont fournis par la société CUNO, les filtres CUNO Delipid et Delipid Plus, les filtres en profondeur de la série CE (CE15, CE20, CE25, CE30, CE35, CE40, CE45, CE50, CE70, CE75) et de la série DE (DE25, DE30, DE35; DE40, DE45, DE50, DE55, DE560, DE65, DE70, DE75) qui sont fournis par la société Millipore corp., les filtres de la série HC (A1 HC, B1HC, COHC) et les filtres Clarigard and Polygard de la société Millipore corp. Comme autre système de filtration on cite également les filtres en profondeurs distribués par ManCel associates (par exemple PR 12 UP, PR12, PR 5 UP) ou les filtres de PALL et de la société SeitzShenk Inc ( par exemple Bio20, SUPRA EKIP, KS-50P). De façon avantageuse, l'étape de clarification peut être réalisée au moyen d'une capsule filtrante comprenant deux membranes de porosité distinctes, ce qui revient à réaliser deux filtrations successives en une seule étape. Les capsules filtrantes Sartopore-2 commercialisées par Sartorius comprenant une membrane de 0,8 µm et une membrane de 0,45 µm conviennent bien pour clarifier le surnageant de culture. Au besoin, on peut également incorporer préalablement une étape de pré-filtration comme indiquée précédemment.

Le surnageant de culture qu'il soit clarifié ou non est en cas de besoin ajusté à un pH compris entre 7 et 8, de préférence entre 7,0 et 7,6. On vérifie également que la conductivité du surnageant est ≤20 mS/cm, de préférence entre 10 et 20 mS/cm et de façon particulièrement préférée entre 14 et 18 mS/cm. Le surnageant de culture est ensuite introduit dans une colonne chromatographique échangeuse de cations contenant les particules de Fractogel ® EMD S03- (type M) et dont les dimensions sont adaptées au volume de surnageant à purifier. La colonne est préalablement équilibrée dans un tampon d'équilibration de faible force ionique (≤ 200 mM). La colonne est ensuite lavée avec un tampon de lavage qui a généralement la même composition que le tampon d'équilibration. On retrouve en sortie de colonne, principalement les impuretés protéiques et les acides nucléiques. On élue ensuite le virus qui a été retenu sur la colonne en utilisant un tampon d'élution dont la force ionique est d'au moins de 400 mM. A titre d'exemple de solutions tampons convenant bien à l'objet de l'invention on cite le tampon Tris 20 mM, Nacl 150mM, pH=7,5 pour équilibrer et laver la colonne, et le tampon Tris 20 mM, Nacl 600 mM, pH=7,5 comme tampon d'élution du virus mais toute autre solution tampon équivalente pourrait également convenir. Les capacités de charge de la colonne étant respectées, on récupère dans l'éluât plus de 70 % de la quantité de virus qui a été initialement introduite dans la colonne, tandis que les quantités d'ADN et de protéines totales présentes dans l'éluât représentent respectivement moins de 5 % et moins de 40 % des quantités initiales d'ADN et de protéines totales qui ont été introduites dans la colonne. Ces résultats sont obtenus notamment dans les cas où les surnageants de culture infectieux sont dépourvus de sérum d'origine animale, ou ne contiennent pas de protéine sérique, ou contiennent uniquement des protéines exogènes d'origine non animale à de faibles concentrations (≤ 15 mg/l).

Les inventeurs ont également montré qu'en combinant une étape de chromatographie par échange de cations sur un support comprenant une matrice de polymethacrylate sur laquelle ont été greffés par liaison de covalence des groupements sulfoisobutyl avec une étape de digestion enzymatique des acides nucléiques, il était encore possible d'abaisser le taux d'ADN résiduel d'environ 1,5 à 2 log₁₀ supplémentaires si bien qu'en combinant ces deux étapes on arrive à éliminer au moins 4,0 log₁₀ d'ADN. A titre de comparaison, en utilisant un procédé d'élimination des acides nucléiques basé sur une digestion enzymatique des acides nucléiques que l'on répète deux fois, on obtient un taux d'élimination d'ADN qui ne dépasse pas 3,5 log₁₀, la deuxième digestion ne permettant pas d'abaisser le taux résiduel d'ADN au-delà de 0,5 à 1 Log₁₀ supplémentaire (voir exemple 2). Comme agent enzymatique de digestion des acides nucléiques, on peut utiliser une ou plusieurs enzymes, de préférence une RNAse et/ou une DNAse, ou un mélange d'endonucléases connus de l'homme de l'art, comme par exemple la Pulmozyme^{™}. Dans le cadre du procédé selon l'invention on utilise généralement la Benzonase^{™}, obtenue de préférence par recombinaison génétique, dans une gamme de concentration généralement comprise entre 1 et 50 U/ml. C'est une endonucléase qui agit en coupant rapidement l'ADN et l'ARN cellulaires sous la forme de tous petits fragments et qui réduit la viscosité du milieu. La température et la durée du traitement enzymatique sont des paramètres facilement maitrisables par l'homme de l'art et dépendent de la concentration initiale de l'endonucléase dans le milieu réactionnel. Pour éviter tout phénomène d'agrégation on peut ajouter à l'éluât chromatographique une très faible quantité d'un agent tensioactif qui est de préférence non ionique, comme le poloxamer 188 (Pluronic F 68) à une très faible concentration. Préalablement à l'étape de traitement enzymatique, on peut éventuellement concentrer l'éluât lorsque le volume est important. L'étape de concentration est généralement réalisée par ultrafiltration sur membrane ayant un seuil de coupure compris entre 100 kDa et 300 kDa, de façon préférée entre 100 kDa et 200 kDa. L'ultrafiltration se caractérise par un flux tangentiel sur la membrane induisant une force qui permet la diffusion des molécules à travers la membrane poreuse. Le flux imposé par une pompe de recirculation se répartit en deux composantes : le flux de recirculation qui assure le balayage (ou flux rétentat) et le flux de filtrat (perméat) qui traverse la membrane. La composition de la membrane peut être, de façon non limitative en cellulose régénérée, en polyethersulffone, en polysulfone ou en des dérivés de ces produits. Elle peut se présenter sous la forme de feuilles plates à l'intérieur de cassettes (notamment pour l'ultra filtration tangentielle) ou de fibres creuses. Les membranes sont notamment commercialisées par Pall sous les dénominations Oméga^{™}, par Millipore sous les dénominations de membranes Biomax^{™}, par Sartorius sous la dénomination de sartocon®. On peut également exercer une contre-pression au niveau du filtrat (permeat) de façon à réduire la pression transmembranaire comme cela est décrit dans WO 2006/108707. Au fur et à mesure que l'éluât traverse la membrane, le volume de l'éluât diminue et le virus ne traversant pas la membrane se concentre. Lorsqu'on ultra filtre l'éluât, on peut diminuer son volume d'un facteur pouvant aller de 1 à 100, voire 150 permettant ainsi d'obtenir le volume final désiré. Cette étape de concentration peut être complétée avec une étape de diafiltration qui permet de modifier la composition du tampon sans pour autant modifier le volume du rétentat. Ceci est conseillé lorsque la composition du tampon dans le rétentat du fait de la diminution de volume de l'éluât n'est plus compatible avec une bonne activité enzymatique de l'endonuclease. On procède alors en ajoutant au flux de recirculation du rétentat, un tampon dont la composition est compatible avec une bonne activité enzymatique de l'endonuclease. On cite à titre d'exemple et sans caractère de restriction des compositions de tampon compatibles avec une bonne activité enzymatique d'une endonucléase comme la Benzonase^{™}: elles contiennent un tampon Tris dans une gamme de concentration en molarité allant de 10 à 50 mM, du MgCl₂ dans une gamme de concentration allant généralement de 1 à 10 mM et éventuellement un autre sel tel que NaCl dans une gamme de concentration allant de 100 mM à 600 mM, le pH de ces solutions tampons se situant dans une gamme de pH allant de 7,0 à 8,0. Comme indiqué précédemment, on peut également traiter directement l'éluât recueilli après l'étape chromatographique en ajoutant du MgCl₂ dans une gamme de concentration allant généralement de 1 à 10 mM puis une endonucléase, telle que la Benzonase^{™} à la concentration désirée.

Pour compléter l'élimination des impuretés protéiques, et éliminer notamment la benzonase, on soumet l'éluât qui a été traité avec l'endonucléase à une étape d'ultracentrifugation sur gradient de saccharose, cette étape d'ultracentrifugation pouvant être répétée une ou plusieurs fois. L'ultracentrifugation permet l'isolement du virus rabique en jouant sur la différence de coefficient de sédimentation entre les différentes entités présentes dans l'éluât: les impuretés protéiques migrent dans les faibles densités de saccharose (≤35 % de sucrose) alors que le virus rabique se situe dans les plus fortes densités de saccharose (≥35 % de sucrose). L'étape d'ultracentrifugation sur gradient de saccharose est généralement réalisée selon la méthode dite du « flux continu » lorsque les volumes à traiter sont importants ou en rotor lorsque les volumes sont relativement faibles. L'ultracentrifugation sur gradient de saccharose se fait habituellement sur « coussins de saccharose » à une température voisine de 5°C. L'éluât qui a été traité avec une endonucléase peut être sous une forme non concentrée ou concentré 10 fois, 20 fois, 50 fois, 100 fois, 150 fois, ou même plus. Le ratio entre le volume total de l'éluât à ultracentrifuger et le volume total du gradient de saccharose se situe habituellement entre 0,5 et 2,0 et de façon préférée entre 0,5 et 1,5. La densité du « coussin » de saccharose de faible densité se situe habituellement dans une gamme allant de 10 à 35 % p/p tandis que la densité du « coussin » de saccharose de forte densité se situe habituellement dans une gamme allant de 40 à 60 % p/p. Le volume de saccharose de faible densité est généralement plus important que le volume de saccharose de forte densité et ce d'autant plus que le volume de l'éluât soumis à l'étape d'ultracentrifugation est important. Habituellement le ratio entre le volume de saccharose de forte densité et le volume de saccharose de faible densité se situe entre 0,3 et 0,7. La durée de l'ultracentrifugation est fonction de la vitesse d'ultracentrifugation qui est en général ≥ 30000 g. Un temps d'ultracentrifugation de 2 heures suffit généralement lorsque la vitesse d'ultracentrifugation est ≥ 65000 g. Après ultracentrifugation, le produit réparti dans le gradient est déchargé à la pompe péristaltique et fractionné. Le taux de saccharose contenu dans chaque fraction est mesuré à l'aide d'un réfractomètre. On mesure aussi dans chaque fraction la quantité de virus rabique sur la base du dosage de la glycoprotéine gpG par ELISA et la quantité de protéines totales par la méthode de Bradford. On établit ainsi le profil de séparation. A l'issue de ces analyses, les fractions du gradient contenant essentiellement le virus rabique sont sélectionnées et rassemblées. On arrive à éliminer au cours de cette étape plus de 85 % et de préférence plus de 90 % des protéines totales, essentiellement les impuretés protéiques puisque le rendement en virus rabique est ≥ 70 % et de préférence ≥ 80 %.

Ainsi, dans le procédé de purification selon l'invention lorsque l'on combine l'étape de clarification du surnageant de culture de cellules infectées par le virus rabique, l'étape de chromatographie par échange de cations sur un support constitué d'une matrice de type polyméthacrylate sur laquelle sont greffés par liaison de covalence des groupements sulfoisobutyl suivie éventuellement d'une concentration et/ou diafiltration par ultrafiltration de l'éluât, l'étape de traitement par une endonuclease de l'éluât et l'étape d'ultracentrifugation sur gradient de saccharose on obtient une composition de virus rabique purifié qui, pour une quantité en virus rabique correspondant à 4,5 UI (sur la base de la mesure de la gpG par ELISA) ou à une dose de vaccin efficace, contient moins de 50 pg d'ADN résiduel, de façon préférée moins de 20pg d'ADN résiduel et de façon encore plus préférée moins de 10 pg d'ADN résiduel (mesuré par qPCR) et contient moins de 40 µg de protéines totales, de façon préférée moins de 20 µg de protéines totales (mesuré par la méthode de Bradford). Habituellement, au moins 70 % des protéines totales sont des protéines virales. Le rendement global en virus purifié est d'au moins 45%, de façon préférée d'au moins 50% et de façon particulièrement préférée d'au moins 60%. Le rendement global en virus purifié est calculé sur la base du ratio entre la quantité de virus présent dans la (les) fraction(s) du gradient recueillie(s) après ultracentrifugation et la quantité de virus initialement présente dans le volume de surnageant viral qui a été traité. Parallèlement, on élimine plus de 4 log₁₀ d'ADN et plus de 95% des protéines totales (Voir tableau I). De façon préférée, le surnageant de culture de cellules infectées par le virus rabique est dépourvu de toute protéine sérique ou, dépourvu de toute protéine exogène d'origine animale, ou même dépourvu de tout produit exogène d'origine animale pour garantir notamment une plus grande sécurité biologique de la composition vaccinale.

A titre d'exemple, le tableau I ci-dessous indique pour chaque étape du procédé de purification du virus rabique à partir d'un surnageant de culture de cellules Vero infectées les quantités de virus récupérées ainsi que les quantités d'ADN et de protéines éliminées.

**Tableau I : Tableau résumant les différentes étapes du procédé de purification du virus rabique avec les rendements associés en virus et les quantités d'ADN et de protéines éliminées.**

| Etape | Quantité de virus récupéré (en %)* | | Quantité de protéines éliminées (en %)** | | Quantité d'ADN éliminé (en log ₁₀)*** | |
|---|---|---|---|---|---|---|
| | à l'étape | global | à l'étape | global | A l'étape | global |
| Clarification | 90 | 90 | ND | ND | ND | ND |
| Chromatographie sur support EMD/SO₃⁻ | 89 | 80 | 60 | 60 | ND | ND |
| Ultrafiltration/diafiltration | 97 | 77 | 18 | 67 | 2,7 | 2,7 |
| Traitement benzonase + Ultra centrifugation sur coussins de sucrose | 70 | 54 | 88 | 96 | 1,6 | 4,3 |

| | | | | | | |
|---|---|---|---|---|---|---|
| *: la quantité de virus rabique est déterminée sur la base du dosage de la glycoprotéine G du virus rabique par ELISA selon la méthode décrite dans l'exemple 1. ** : la quantité de protéines totales est mesurée par la technique de Bradford *** : la quantité d'ADN est mesurée par qPCR selon la méthode décrite dans l'exemple 1. ND : Non déterminé | | | | | | |

Le virus rabique purifié recueilli après l'étape d'ultracentifugation est généralement sous une forme trop concentrée que l'on dilue souvent pour éviter la formation d'agrégats viraux lors du stockage. On utilise habituellement comme tampon de dilution une tampon phosphate à un pH d'environ 8, éventuellement additionné d'une solution saline comme par exemple une solution de chlorure de sodium. Grâce au procédé selon l'invention, la suspension de virus rabique purifiée obtenue peut être utilisée comme vaccin à virus vivants ou atténués ou comme vaccin à virus inactivés. Lorsque le vaccin est destiné à la médecine humaine, on inactive généralement la suspension de virus purifiée.

### Etape d'inactivation virale

Le procédé de purification du virus rabique tel que décrit dans l'invention se termine par une étape d'inactivation virale lorsque le virus est destiné à la fabrication d'un vaccin inactivé. L'inactivation virale peut être réalisée au moyen d'agents chimiques bien connus de l'homme du métier tels que le formaldéhyde, le glutaraldéhyde, ou la β propiolactone. On peut également utiliser le procédé d'inactivation tel que décrit dans WO 2005/093049 qui consiste à mettre en contact la solution virale purifiée avec un composé hydrophobique photo activable et à exposer ce mélange à la lumière. Parmi les composés hydrophobiques photo activables on cite l'azidobenzene, 1-azidonaphthalene, 4-azido-2- nitro-1-(phenylthio) benzene, 1-azido-4-iodobenzene, 1-azido-5- iodonaphthalene, 3-phenyl-3H-diazirene, 3-phenyl-3-(trifluoromethyl)-3H- diazirene, 3-(3-iodophenyl)-3-(trifluoromethyl)-3H-diazirene, 1-azidopyrene, adamantanediazirene, 12- (4-azido-2-nitrophenoxy)-stearic acid, w- (m-diazirinophenoxy) fatty acid, 12 [ (azidocarbonyl) oxy] stearic acid, 12-azidostearic acid, 11- (3-azidophenoxy) undecanoic acid or w- (m- diazirinophenoxy) undecanoic acid ou le 1,5-iodonaphtyl azide. De façon préférée, on utilise la β propiolactone (BPL) car c'est à la fois un agent d'inactivation virale et un agent alkylant entrainant des coupures dans l'ADN. Il peut donc contribuer également à la réduction du taux d'ADN résiduel et à l'inhibition de son activité biologique. L'inactivation du virus rabique est réalisée au moyen d'une solution de β propiolactone dilué entre 1/3500 et 1/4000 (concentration volumique finale dans la solution contenant le virus purifié) à une température d'environ 12°C. Plus la concentration en β propiolactone est faible et plus le temps nécessaire à l'inactivation du virus est important. Généralement l'inactivation du virus se fait dans un intervalle de temps allant de 12 h à 48 h. On neutralise l'activité de la β propiolactone par simple chauffage de la solution à une température d'environ 37°C pendant environ 2 h. On vérifie ensuite que le pH de la solution est > 7,0. Au besoin, on rectifie le pH au moyen d'une solution de soude diluée ou alternativement, avant le traitement à la β propiolactone, la solution contenant le virus purifié est tamponnée au moyen d'une solution à base d'un tampon phosphate, pH ∼ 8, ce qui évite toute acidification de la solution lors de l'hydrolyse de la β propiolactone.

La suspension virale purifiée selon le procédé de l'invention est en général conservée dans un tampon de conservation comme par exemple dans un tampon Tris ou un tampon phosphate. Bien que l'on puisse mélanger directement la suspension virale purifiée avec le tampon de conservation, on procède généralement à une étape de diafiltration par ultrafiltration dans un tampon de conservation, au besoin complétée par une étape de concentration si le virus purifié n'est pas suffisamment concentré. Lorsqu'une étape d'ultrafiltration est mise en oeuvre, on utilise une membrane dont le seuil de coupure est généralement compris entre 5 kDa et 100 kDa, de façon préférée entre 8 kDa et 50 kDa. Le stock de virus purifié ainsi préparé est finalement stérilisé par filtration sur membrane ayant une porosité ≤ 0,2 µm, puis conservé à +5°C environ ou, de façon préférée sous une forme congelée avant d'être réparti sous la forme de vaccins uni-doses ou multi-doses. On peut également inclure une étape supplémentaire de lyophilisation des préparations vaccinales. Dans ce cas, la composition du tampon de conservation est choisie de telle manière qu'il puisse être lyophilisé. Le rendement global en virus purifié inactivé calculé ici sur la base du ratio entre la quantité de virus présent dans le stock de virus purifié obtenu après l'étape ultime de filtration stérilisante et la quantité de virus présente initialement dans le volume de surnageant viral (avant clarification) qui a été traité est encore d'au moins 40 % ce qui témoigne de l'intérêt industriel d'un tel procédé de purification.

En définitive, un vaccin contenant du virus rabique purifié peut être produit avec un très bon rendement et avec une très grande pureté en utilisant un procédé selon lequel les étapes :
- de production d'un lot de cellules à partir d'une banque de cellules, généralement à partir d'une banque cellulaire de travail,
- de production du virus rabique après infection du lot de cellules, et
- de purification du virus rabique à partir du surnageant de culture de cellules infectées,
sont toutes réalisées en utilisant des produits exogènes d'origine non animale.

Grâce à ce procédé, le vaccin obtenu est dépourvu de tout produit exogène d'origine animale.

Par conséquent, l'invention a également pour objet un procédé de fabrication d'un vaccin contre la rage selon lequel :
a) on produit un lot de cellules,
b) on infecte le lot de cellules avec du virus rabique,
c) on purifie le virus rabique à partir du surnageant de culture de cellules infectées selon le procédé de l'invention,
d) on mélange la suspension de virus purifié dans un tampon de conservation et,
e) on répartit le mélange sous la forme de vaccins unidoses ou multidoses.

Selon une variante du procédé de fabrication du vaccin, le tampon de conservation est un tampon de lyophilisation. Dans ce cas, on mélange la suspension de virus purifié avec le tampon de lyophilisation, on répartit le mélange sous la forme de vaccins unidoses ou multidoses puis on lyophilise les doses vaccinales.

Selon une variante préférée du procédé de fabrication du vaccin, toutes les étapes du procédé sont réalisées en utilisant des produits d'origine non animale. Dans ce cas le vaccin obtenu est dépourvu de tout produit exogène d'origine animale et offre ainsi une plus grande sécurité biologique.

Le virus rabique contenu dans le vaccin obtenu selon le procédé de l'invention peut être inactivé ou atténué. L'analyse densitométrique du profil électrophorétique d'un échantillon de vaccin obtenu après électrophorèse sur gel de polyacrylamide et révélation au bleu de Coomassie montre en effet que plus de 70 % des protéines sont d'origine virale. On retrouve essentiellement les 5 protéines du virus rabique (c.a.d. la glycoprotéine G, la nucléoprotéine N, la phosphoprotéine P, la protéine de matrice M, la RNA polymerase L) sur un gel de polyacrylamide (SDS-PAGE) réalisé en présence de 2-mercaptoethanol.

De plus, l'analyse granulométrique d'un échantillon de vaccin au moyen de l'appareil zetasizer Nano ZS (Malvern Instruments) qui mesure le mouvement brownien des particules sur la base de la diffusion « quasi élastique » de la lumière (Dynamic Light scattering) montre l'existence d'une seule population de particules comprise entre 100 et 300 nm avec une valeur moyenne à 180 nm qui correspond à la taille moyenne du virus rabique. L'analyse d'un échantillon de vaccin par microscopie électronique révèle également la présence de particules virales ayant la forme classique d'obus. Le vaccin obtenu selon le procédé de l'invention est donc sous la forme d'une suspension homogène de virus rabiques purifiés entiers caractérisée en ce que l'analyse granulométrique au moyen de l'appareil zetasizer Nano ZS montre l'existence d'un seul pic compris entre 100 et 300 nm environ. Cette suspension homogène de virus rabiques purifiés avec les caractéristiques de pureté telles que décrites est obtenue en mettant en oeuvre le procédé de purification tel que décrit dans l'invention.

Le test officiel admis par l'OMS pour évaluer l'efficacité du vaccin antirabique est le test NIH (décrit notamment dans la monographie de la Pharmacopée Européenne n° : 216 concernant les vaccins rabiques préparés à partir d'une culture de cellules et destinés à la médecine humaine). Pour être efficace une dose de vaccin doit contenir au moins 2,5 UI selon le test NIH. L'administration d'une dose efficace de vaccin antirabique (c.a.d. au moins 2,5 UI selon le test NIH) par voie intramusculaire chez l'homme selon les protocoles de vaccination ou de séro-vaccination habituellement préconisés induit le développement d'une immunité protectrice. Par ailleurs, on a mis en évidence que lorsque la quantité de virus rabique contenue dans une dose de vaccin obtenu selon le procédé de l'invention est d'au moins 4,5 UI sur la base de la mesure de la glycoprotéine G par ELISA, cette quantité correspond à au moins une dose efficace de vaccin (c'est-à-dire contient au moins 2,5 UI selon le test NIH).

Le test NIH est un test d'épreuve réalisé chez la souris selon lequel on détermine la quantité d'un vaccin test nécessaire pour obtenir la même protection des souris contre les effets létaux d'une préparation de virus rabique préalablement titrée et administrée par voie intracérébrale que celle que l'on observe avec une quantité d'une préparation d'un vaccin antirabique de référence. La préparation du vaccin antirabique de référence est calibrée en unités internationales (UI). L'unité internationale (UI) est l'activité contenue dans une quantité déterminée d'un standard international. L'équivalence en UI du standard international est établie par l'OMS. Après avoir vérifié que les paramètres de contrôle du test d'efficacité sont bien respectés, on détermine alors à partir des valeurs de la dose protectrice 50% du vaccin testé et de la préparation de référence (calibré en UIs), le nombre d'UIs contenus dans le vaccin test. Il doit être d'au moins 2,5 UI par dose pour être efficace dans la vaccination.

La dose efficace du vaccin qui est injectée par voie intra musculaire chez l'homme est généralement contenue dans un volume de 0,5 à 1 ml d'une suspension liquide prête à l'emploi ou obtenue par simple décongélation ou encore obtenue extemporanément par reconstitution d'un lyophilisat avec un solvant.

Les protocoles vaccinaux utilisés pour protéger l'homme contre la rage sont bien connus et diffèrent selon qu'il s'agit d'une vaccination préventive ou curative. Habituellement, dans le cas d'une vaccination préventive le protocole de primo vaccination comprend deux ou 3 injections intra musculaire d'une dose efficace de vaccin. Les rappels vaccinaux se font ensuite, à intervalles réguliers par administration d'une seule dose efficace de vaccin. Dans le cas d'une vaccination curative, les protocoles de vaccination diffèrent selon que l'individu exposé au virus rabique a déjà été vacciné ou non et selon les pays. Le protocole de vaccination habituellement préconisé chez les sujets non immunisés ou mal immunisés parallèlement à l'administration d'un sérum antirabique comporte 5 injections successives d'une dose efficace de vaccin par voie intra musculaire sur une période de 1 mois suivie d'un rappel à 3 mois. Le nombre d'injections est réduit à 3, voire à un lorsque les sujets exposés au virus ont reçu au préalable une vaccination préventive complète. D'autres protocoles de vaccination dans le cadre de la vaccination curative chez des sujets non immunisés ou mal immunisés permettant de réduire le nombre d'injections et/ou de quantité d'antigène vaccinal administré peuvent être utilisés dans le cadre de la présente invention. Il s'agit notamment du protocole «Zagreb» qui comporte 4 injections d'une dose efficace de vaccin par voie intra musculaire (avec 2 injections en des sites différents se faisant au jour J0, suivi d'une injection à J7 puis à J21) ou de protocoles de vaccination par voie intra-dermique.

La présente invention sera mieux comprise à la lumière des exemples suivants qui servent à illustrer l'invention sans pour autant en limiter le contenu.

### Exemple 1 : comparaison de différents supports chromatographiques dans le procédé de purification du virus rabique

### 1-1) Production du virus rabique sur cellules VERO en milieu sans sérum

Les cellules de la lignée VERO, après les avoir adaptées à des conditions de culture en milieu sans sérum comme décrit dans WO 01/40443, ont été transférées dans un biogénérateur de 10 à 20 litres contenant des microporteurs de cytodex 1 dans le milieu VP SFM (Invitrogen). Après une période de culture de 3 à 4 jours à 37°C en maintenant un pH d'environ 7.2 ± 0.2 , une saturation en oxygène de 25 % ± 10 % et en soumettant le milieu à une faible agitation, les cellules ont été infectées avec du virus rabique à une multiplicité d'infection de 0,01 dans un milieu d'infection virale contenant du milieu VP SFM (Invitrogen). Dans le cas des essais qui ont été réalisés avec le support chromatographique Fractogel EMD-SO3⁻, la production du virus rabique a été réalisée à l'échelle de 150 litres en utilisant des biogénérateurs de 150 litres. On a récolté les surnageants de culture des cellules infectées aux jours J7 (R1); J11 (R2) et J15 (R3). Après chaque récolte, on a réintroduit du milieu d'infection virale neuf.

### 1-2) Clarification et analyse des récoltes

L'étape de clarification a été réalisée en utilisant deux filtrations frontales successives ; la première en utilisant un pré-filtre en polypropylène de 8 µm (Sartopure PP2, SARTORIUS) qui élimine les quelques microporteurs aspirés lors de la récolte, les cellules Vero décollées des supports et les débris cellulaires de taille importante ; la deuxième en utilisant un filtre PES, composé de la combinaison de deux filtres 0,8 µm et 0,45 µm (Sartopore 2, SARTORIUS) qui élimine les agrégats.

La quantité de virus rabique présente dans les récoltes clarifiées a été déterminée en mesurant la quantité de glycoprotéine G (gpG) mesurée par la méthode ELISA suivante :

On a réparti dans les puits d'une microplaque ELISA environ 0,12µg/100µl d'une solution d'un anticorps monoclonal 1112-1 anti-gpG (dont les caractéristiques sont décrites dans Journal of Clinical investigation (1989), volume 84, pages 971 à 975) préalablement diluée dans un tampon de « coating » (tampon carbonate/bicarbonate 0,2M, pH 9,6). Après une incubation d'une nuit en chambre froide, suivie de plusieurs lavages avec un tampon de lavage (tampon phosphate additionné de Tween 20, 0,05 %), on a distribué dans chaque puits 100 µl d'un tampon de saturation (tampon phosphate additionné de sérum albumine bovine à 1 %). Après une incubation d'une heure à 37°C, suivie de plusieurs lavages, on a réalisé une gamme de dilution de chaque échantillon à tester dans un tampon de dilution (tampon phosphate additionné de Tween 20 à 0,05 % et de sérum albumine à 0,1 %). En parallèle, on a réalisé dans chaque microplaque une gamme de dilution d'un étalon de référence, qui a été calibré vis-à-vis de la référence internationale du NIBSC (par exemple PISRAV). Après une nouvelle incubation d'une heure à 37°C, suivie de plusieurs lavages, on a distribué dans chaque puits 100 µl d'une solution d'un anticorps monoclonal D1 de souris anti-gpG (dont les caractéristiques sont décrites dans Biologicals (2003), volume 31, pages 9 à 16) qui a été biotinylé et utilisé après dilution au 1/5000 dans le tampon de dilution. Les plaques ont été laissées 1 heure à 37°C puis lavées à plusieurs reprises avant de distribuer dans chacun des puits 100 µl d'une solution de streptavidine couplée à la peroxydase (Southern Biotechnology Associates) préalablement diluée au 1/15000 dans le tampon de dilution. Après une nouvelle incubation d'une heure à 37°C suivie de plusieurs lavages on a distribué dans chaque puits 100 µl d'une solution d'un tampon citrate 0,05 M, pH 5 contenant le substrat de révélation (O-Phenylènediamine). Après un temps d'incubation de 30 minutes à température ambiante à l'abri de la lumière on a stoppé la réaction de révélation en ajoutant 50 µl/puits d'une solution d'H₂SO₄ 2N. La lecture spectrophotométrique des microplaques a été réalisée à deux longueurs d'onde (492 nm et 620 nm). La densité optique mesurée est la différence entre les deux lectures pour tenir compte de l'absorption du plastique. Le calcul de l'activité relative a été effectué par la méthode des droites parallèles selon les recommandations de la Pharmacopée Européenne. Le titre en virus rabique de l'échantillon est basé sur la détermination de la concentration en glycoprotéine G du virus rabique qui est exprimée en UI/ml par rapport à la référence.

La quantité de protéines totales présente dans les récoltes clarifiées a été mesurée en utilisant la méthode classique de Bradford commercialisé sous la forme d'un Kit par Biorad (Ref: 500-0006)

La quantité d'ADN présente dans les récoltes clarifiées a été mesurée par q-PCR. Le protocole opératoire est similaire à celui qui est décrit par Lebron J.A. et coll. dans Developments in Biologicals (2006), vol 123, pp35-44. Après avoir extrait au moyen du kit commercial DNA Extractor de Wako Pure Chemicals l'ADN résiduel des récoltes clarifiées, on a introduit dans chaque échantillon une quantité fixe d'un ADN exogène qui sert de contrôle interne de l'amplification par PCR. En parallèle, on a préparé un échantillon d'ADN génomique provenant de cellules Vero lysées par congélations/décongélations successives, traitées par la suite à la RNase A à raison de 2 mg de RNase A pour 2,5.10⁵ cellules, puis enfin que l'on a ensuite purifié en utilisant le kit QIAamp Virus BioRobot 9604 (QIAGEN). L'ADN purifié a été quantifié par spectrophotométrie à 260 nm. A partir de cet ADN purifié (ADN standard) on a réalisé une gamme d'étalonnage en réalisant des dilutions de raison 10. Les échantillons provenant des récoltes clarifiées ainsi que les échantillons de la gamme d'étalonnage ont été soumis ensuite à un cycle d'amplification par PCR après avoir rajouté pour chaque essai la sonde fluorescente Alpha -MPr3, les deux amorces, le prémélange 2X QuantiTect Probe Master Mix (Qiagen) dans un volume d'eau dépourvu de toute nucléase (qsp 50 µl). Le cycle d'amplification a été réalisé en utilisant l'appareil Light Cycler 480 (Roche Applied Science) en utilisant le programme 95°C, 15 min.; 40 cycles comprenant deux étapes 95°C, 15 sec.; 60°C, 60 sec. On a ensuite calculé par interpolation la quantité d'ADN résiduel extrait des récoltes clarifiées sur la base de la mesure de la fluorescence observée par rapport à la gamme d'étalonnage établie avec l'ADN standard. La quantité d'ADN résiduel a été ajustée par un facteur correctif correspondant au rendement de charge mesuré pour l'échantillon par l'intermédiaire de la quantification de l'ADN exogène. En général, les concentrations en glycoprotéine G (correspondant au titre en virus rabique), en protéines totales et en ADN dans les récoltes clarifiées se situaient dans des gammes allant de 1 à 3 UI/ml pour la glycoprotéine G, de 50 à 100 µg/ml pour les protéines totales et de 5 à 50 ng/ml pour l'ADN.

### 1-3) Mesure des performances de différents supports chromatographiques pour leur capacité à éliminer les acides nucléiques et à retenir le virus rabique

L'élimination de l'ADN cellulaire dans les récoltes clarifiées a été évaluée sur les supports chromatographiques suivants :

### Supports anioniques

- Fractogel ® EMD TMAE (Merck) (échangeur d'anions fort)
- Fractogel ® EMD DEAE (Merck) (échangeur d'anions faible)
- Membrane chargée positivement Sartobind ® Q (Sartorius)
- Membrane chargée positivement Mustang ® Q (Pall)

### Support cationique

- Fractogel ® EMD S03- (Merck) (échangeur de cations fort)

Des essais préliminaires ont été réalisés sur la membrane chargée positivement Sartobind® de façon à identifier la meilleure concentration en sel pour éluer le virus par chromatographie échangeuse d'anions. Ces essais ont montré que le virus était élue tout au long du gradient de sel y compris à 1M. Ceci était également vrai pour les autres supports anioniques qui ont été testés. Les profils d'élution obtenus nous ont conduits à choisir une forte concentration en NaCl pour éluer le maximum de virus. L'ADN commençant à être élué à partir de 350-400 mM, le compromis a été de choisir une concentration en NaCl de 450mM.

Les supports anioniques ont été équilibrés en tampon TRIS 20 mM, NaCl 150 mM pH 7,5. Les récoltes clarifiées ont été ensuite mises en contact avec les différents supports étudiés. Les supports anioniques ont été ensuite rincés en tampon Tris 20 mM, NaCl 150mM, pH 7,5. Le virus a été ensuite décroché du support au moyen d'un tampon d'élution Tris 20 mM, Nacl 450 mM, pH 7,5.

Les essais sur colonne de Fractogel ® ont été réalisés en utilisant la récolte clarifiée R1. Entre 35 et 50 UI de virus rabique et entre 13 et 25 µg d'ADN ont été injectés par ml de gel.

Les essais sur membrane ont été réalisés en utilisant la récolte clarifiée R2. 13 UI de virus rabique et entre 350 et 450 ng d'ADN ont été injectés par cm² de membrane.

Les conditions opératoires utilisées pour la chromatographie échangeuse de cations sur Support Fractogel® EMD S03- ont été également optimisées. Le support Fractogel® EMD S03- a été équilibré en utilisant un tampon Tris 20 mM, NaCl 150 mM, pH =7,5. Les essais sur support Fractogel® EMD S03- ont été réalisés en utilisant la récolte clarifiée R1. On a contrôlé la conductivité du milieu (entre14 mS/cm et 18 mS/cm) et le pH (entre 7,5 et 7,7). Environ 50 UI (sur la base du dosage de la glycoprotéine G) de virus rabique ont été injectés par ml de gel. Après avoir récolté le filtrat pour mesurer la quantité de virus qui ne s'est pas fixé sur la colonne, le support a été ensuite lavé en tampon Tris 20mM, NaCl 150mM, pH= 7,5. Le virus a été ensuite élué dans le tampon Tris 20mM, NaCl 600 mM, pH=7,5 avec récupération du pic viral en une fraction indépendante. Le support a été finalement régénéré en tampon Tris 20mM, NaCl 1M, pH=7,5. Les résultats obtenus figurent dans le tableau II ci-dessous :

**Tableau II : Performances des différents supports chromatographiques**

| Caractéristiques du support chromatographique | Rend^{t}, en virus | Titre ADN Résiduel (ng/ml) | quantité ADN éliminé (en %). |
|---|---|---|---|
| Mustang Q | 47 % | 4** | 93% |
| Sartobind Q | 50% | 1** | 98% |
| Fractogel EMD-TMAE | 54% | 196** | 48% |
| Fracto gel EMD-DEAE | 70% | 161** | 73% |
| Fracto gel EMD-S03⁻ | 89% | 4,15*** | 99,7%*** |

| | | | |
|---|---|---|---|
| *: le titre en virus rabique a été déterminé sur la base de la mesure de la glycoprotéine G par ELISA ** : le titre en ADN résiduel a été mesuré par q PCR directement sur le produit recueilli *** : le titre en ADN résiduel a été mesuré par qPCR après concentration de l'éluât d'un facteur 6 par ultrafiltration. | | | |

Les supports membranaires chargés positivement (Sartobind® (Sartorius) ou Mustang® Q (Pall)) facilitent l'élimination de l'ADN (plus de 90% de l'ADN est éliminé dans la fraction recueillie) mais par contre les rendements en virus récoltés sont relativement faibles (≤ 50%). Concernant les supports à base de gels chargés positivement (Fractogel® EMD TMAE (Merck), Fractogel® EMD DEAE (Merck) on observe plutôt la tendance inverse, à savoir un meilleur rendement en virus récolté (≥ 50%) mais au détriment de l'élimination de l'ADN (73 % de l'ADN est éliminé dans le meilleur des cas). Par contre, lorsque l'on utilise le support cationique Practogel® EMD SO3⁻, on observe à la fois une très bonne élimination de l'ADN (plus de 95 % de l'ADN est éliminé dans la fraction recueillie) et un très bon rendement en virus récolté (≥ 70 %). Les performances chromatographiques du support cationique Fractogel® EMD SO3⁻ sont donc meilleures que celles que l'on constate avec les supports chromatographiques anioniques.

### 1-4) Mesure des performances chromatographiques de différents supports chromatographiques cationiques

Les tests précédents ayant montré que le support chromatographique échangeur de cation fort avait de meilleurs performances que les supports anioniques, nous avons cherché à déterminer les caractéristiques du support chromatographique échangeur de cations fort qui donne les meilleurs performances en testant différents supports chromatogarphiques échangeurs de cations forts se distinguant notamment par leur matrice et leurs groupements (ligands). Les caractéristiques des différents supports testés sont décrites ci-dessous :
- Membrane chargée négativement Sartobind® S (Sartorius) : membrane cellulosique sur laquelle ont été greffés des groupements (ligands) d'acide sulfonique
- Membrane chargée négativement Mustang^{™} S (Pall) : membrane de polyethersulfone sur laquelle ont été greffés des groupements (ligands) d'acide sulfonique
- Gel Capto^{™} S (GE Healthcare): gel dont la matrice est à base d'agarose sur laquelle ont été greffés des groupements (ligands) sulfo ethyl par l'intermédiaire de bras espaceurs à base de dextran.
- Gel SP Sepharose XL (GE Healthcare) : gel dont la matrice est à base d'agarose sur laquelle ont été greffés des groupements (ligands) sulfo propyl par l'intermédiaire de bras espaceurs à base de dextran.
- Toyopearl^{®} SP-650 C (Tosoh) : résine dont la matrice est à base de polymethacrylate sur laquelle ont été greffés des groupements (ligands) sulfo propyl.
- Fractogel® EMD SO₃⁻ (Merck): gel dont la matrice est à base de polymethacrylate sur laquelle ont été greffés des groupements sulfoisobutyl par l'intermédiaire de bras espaceurs constitués de chaînes polymériques obtenues par polymérisation du monomère ayant pour formule chimique : CH₂=CH-CO-NH-C(CH₃)₂-CH₂-SO₃-

Les gels dont les ligands ont été greffés sur la matrice par l'intermédiaire de bras espaceurs ont généralement une structure dite "tentaculaire" qui facilite en général l'interaction ligand/proteine.

Différents paramètres ont été évalués pour la chromatographie sur membrane Sartobind® S (Sartorius) (volume de support : 7 ml) : le pH de la récolte clarifiée injectée qui se situait dans une gamme allant de 7,5 à 8,0, la conductivité de la récolte clarifiée injectée qui se situait dans une gamme allant de 4,5 mS/cm à 17,9 mS/cm et la charge virale injectée qui se situait dans une gamme allant de 4 UI/ml à 11 UI/ml de support. Quels que soient les paramètres étudiés, les rendements en virus obtenus ont toujours été ≤ 35 %.

Dans le cas de la chromatographie sur membrane Mustang^{™} S (Pall) (volume du support : 10 ml), le pH et la conductivité de la récolte clarifiée étaient respectivement de 7,5 et de 14 mS/cm tandis que la charge virale injectée était de 32 UI/ml de support. Le rendement en virus obtenu a été très faible (≤ 10 %).

On a appliqué le même protocole opératoire que celui qui a été utilisé pour la chromatographie sur support Fractogel® EMD SO3⁻ pour effectuer les autres chromatographies sur gel (Voir paragraphe 1-3). La seule distinction concerne la régénération du gel Toyopearl^{®} SP-650 C qui se fait dans un tampon Tris 20mM, NaCl 2M pH 7,5. On a utilisé les mêmes conditions de pH et de conductivité que celles de la chromatographie sur Fractogel® EMD SO₃⁻. On a respecté également les limites de capacité de charge des différents supports chromatographiques lors de l'injection de la charge en virus contenu dans le surnageant clarifié

Hormis le support Fractogel® EMD SO₃⁻ où l'on retrouve dans le filtrat environ 5% de la quantité totale de virus injecté, les quantités en virus qui ont été retrouvées dans les filtrats des chromatographies réalisées sur les 3 autres types de supports (Gel Capto^{™} S, Gel SP Sepharose XL et gel Toyopearl^{®} SP-650 C) représentent entre 80 et 99% de la quantité totale de virus injecté. Les rendements en virus obtenus dans les éluât sont par conséquent très faibles (<10%).

La structure des ligands et de la matrice dans les supports chromatographiques échangeur de cations forts jouent donc un rôle important puisque seul un support comprenant une matrice de polymethacrylate sur laquelle ont été greffés des groupements sulfoisobutyl permet d'obtenir un éluât dans lequel on récupère au moins 70% du virus rabique. En conclusion, sur l'ensemble des supports chromatographiques anioniques et cationiques testés, le support Fractogel® EMD SO₃⁻ (Merck) est le seul qui présente des performances qui soient réellement exploitables au plan industriel puisque le rendement en virus rabique est ≥ 70 % tandis qu'on élimine plus de 95 % de l'ADN.

### Exemple 2 : Intérêt de la combinaison d'une étape de chromatographie sur support Fractogel® EMD SO₃⁻ avec une étape de traitement à la benzonase dans le procédé de purification du virus rabique.

Pour évaluer l'intérêt de cette association, on a comparé cette méthode à une méthode de purification du virus rabique par double traitement à la benzonase. Le traitement à la benzonase est utilisé classiquement pour éliminer les acides nucléiques se trouvant dans un produit biologique. Lorsqu'on répète ce traitement enzymatique on augmente encore l'élimination de l'ADN.

On a comparé un procédé de purification du virus rabique qui utilise un « double traitement à la benzonase » avec le protocole de l'invention selon lequel on a combiné une étape de chromatographie échangeuse de cations sur support Fractogel® EMD SO₃⁻ avec un traitement à la benzonase.

Dans le cas du double traitement à la benzonase (UF-Bz-UF-Bz) le protocole de purification qui a été utilisé correspond à celui qui est décrit dans le Tableau III ci-après :

**Tableau III - Purification du virus rabique par double traitement à la benzonase (UF-Bz-UF-Bz).**

| | |
|---|---|
| Récolte clarifiée 0,45 µm | |
| | |
| Concentration / Dialfiltration par Ultrafiltration Concentration ≈ 20 x C | |
| | |
| Ajustement MgCl₂ 2mM | |
| | |
| Benzonase 15U/ml de récolte | |
| | |
| Concentration / Dialfiltration par Ultrafiltration Concentration ≈ 5 x C | |
| | |
| Ajustement MgCl₂ 2mM | |
| | |
| Benzonase ® 15 U/ml de récolte | |
| | |
| Ultracentrifugation sur gradient de saccharose | |

Dans le cas du traitement combinant une chromatographie échangeuse de cations sur support Fractogel® EMD SO₃⁻ et un traitement à la benzonase (CEX-UF-Bz) le procédé de purification qui a été utilisé correspond à celui qui est décrit dans le Tableau IV ci-après :

**Tableau IV**

| Purification du virus rabique par combinaison d'une chromatographie échangeuse de cations sur support fractogel^{®} EMD SO₃⁻ et traitement à la benzonase (CEX-UF-Bz). | |
|---|---|
| Récolte clarifiée 0,45 µm | |
| | |
| Chromatographie Fractogel EMD SO₃⁻ | |
| | |
| Concentration / Dialfiltration par Ultrafiltration Concentration ≈ 100 x C | |
| | |
| Ajustement MgCl₂ 2mM | |
| | |
| Benzonase ® 15 U/ml de récolte | |
| | |
| Ultracentrifugation sur gradient de saccharose | |

Dans les deux cas, le procédé de purification a été réalisé en partant d'une même récolte R1 clarifiée d'un volume d'environ 20 litres qui a été divisée en deux parties égales.

Dans le cas du procédé UF-Bz-UF-Bz les deux concentrations ont été réalisées sur membrane PES Medium Screen 100KDA (PALL) combinées à une diafiltration en tampon Tris 20 mM, NaCl 150 mM, pH = 7,5 par ultrafiltration, la première étape d'ultrafiltration aboutissant à une réduction d'un facteur d'environ 20 le volume de la récolte clarifiée, tandis que la deuxième ultrafiltration a entrainé une réduction d'un facteur global d'environ 100 le volume de la récolte clarifiée. Avant chaque traitement à la benzonase, on a rajouté une solution de MgCl₂ de sorte que la concentration dans le rétentat était 2 mM. Le traitement à la benzoriase a été réalisé en ajoutant 15 U/ml de récolte brute au milieu réactionnel et en laissant le milieu réactionnel pendant 2 heures à température du laboratoire.

Dans le cas du procédé CEX- UF-Bz, l'étape chromatographique a été réalisée selon les modalités décrites dans le paragraphe 1-3. L'éluât contenant le virus purifié a été ensuite concentré d'un facteur d'environ 5 et diafiltré puis traité à la benzonase en utilisant le même protocole que celui qui a été utilisé dans le procédé UF-Bz-UF-Bz.

Dans les deux procédés, l'étape d'ultracentrifugation a été réalisée sur coussins de sucrose 34-60 % avec un rotor 45 type Ti à 21000 rpm pendant 2 h à +5°C. Les fractions du gradient contenant le virus purifié ont été recueillies, rassemblées puis analysées pour leur contenu en ADN, en virus et en protéines totales.

Le Tableau V, ci-après, indique aux différents stades de purification les titres en ADN, en gpG, et en protéines totales, obtenus en fonction du procédé utilisé.

**Tableau V : Bilan des deux procédés de purification**

| Stade purification | paramètres | Proc. UF-Bz-UF-Bz | Proc. CEX-UF-Bz |
|---|---|---|---|
| Récolte clarifiée | volume | 9620 ml | 9620 ml |
| | Titre ADN (ng/ml) | 270 | |
| | Titre gpG (UI/ml) | 1,7 | |
| | ADN/gpG | 713 x 10³ pg/4,5 UI | |
| UF-Bz-UF | volume | 100 ml | |
| | Titre ADN (pg/ml) | NT | |
| | Titre gpG (UI/ml) | 106,6 UI/ml | |
| CEX-UF | volume | | 100 ml |
| | Titre ADN (pg/ml) | | NT |
| | Titre gpG (UI/ml) | | 113 UI/ml |
| Après UC | volume | 800 ml | 800 ml |
| | Titre ADN (pg/ml) | 1,1 x 10³ | <0,1 |
| | Titre gpG (UI/ml) | 9,7 | 8,46 |
| | Protéines totales/gpG | 36 µg/4,5 UI | 24 µg/4,5 UI |
| | Titre ADN/gpG | 510 pg/4,5 UI | <50 pg/4,5 UI |
| Log 10 réduction ADN totale | | 3,47 | >4,5 |
| Rendement en virus* | | 63% | 55% |

| | | | |
|---|---|---|---|
| UF/Bz/UF : correspond au rétentat obtenu après la deuxième ultrafiltration et juste avant le deuxième traitement à la benzonase dans le protocole UF-Bz-UF-Bz. CEX/UF : correspond au rétentat après obtenu après l'étape d'ultrafiltration et juste avant le traitement à la benzonase dans le protocole CEX- UF-Bz Après UC : correspond au stade où les fractions du gradient contenant le virus purifié ont été rassemblées après ultracentrifugation et après avoir ajusté le volume total de telle sorte qu'il soit 12,5 fois plus concentré que le volume de la récolte clarifiée. * : le rendement en virus est calculé sur la base des titres ELISA gpG | | | |

Les résultats du tableau V montrent que la combinaison d'une chromatographie sur support Fractogel® EMD SO₃⁻ suivi d'un traitement à la benzonase (procédé CEX-UF-Bz) est bien plus efficace dans l'élimination de l'ADN qu'un double traitement à la benzonase (procédé UF-Bz-UF-Bz). On arrive à réduire d'au moins 1 log₁₀ supplémentaire la quantité d'ADN résiduelle en utilisant le procédé CEX-UF-Bz. Ces résultats ont également été confirmés à des échelles de volumes différents.
On note également que le procédé CEX-UF-Bz élimine également d'une façon plus efficace les protéines contaminantes que le procédé UF-Bz-UF-Bz puisque l'on retrouve presque deux fois moins de protéines totales par unité de virus (exprimée sous la forme d'UI de gpG) (5,4 µg dans le procédé CEX-UF-Bz au lieu de 8 µg par UI de gpG dans le procédé UF-Bz-UF-Bz. Ceci résulte du fait que plus de 65 % des protéines sont éliminées lors de l'étape de chromatographie sur support Fractogel® EMD SO₃⁻. Ces résultats montrent que la combinaison d'une étape de chromatographie sur support Fractogel® EMD SO₃⁻ avec une étape de traitement à la benzonase dans le procédé de purification du virus rabique exerce une action conjuguée sur l'élimination de l'ADN dans les récoltes clarifiées de virus rabique qui est bien supérieure à l'effet observé lors d'un double traitement à la benzonase.

## Revendications

1. Un procédé de purification du virus rabique comprenant une seule étape de chromatographie par échange d'ions, la dite étape étant une chromatographie par échange de cations selon laquelle :
a) on met en contact le surnageant d'une culture de cellules infectées par ce virus avec un support de chromatographie échangeur de cations comprenant une matrice de polymethacrylate sur laquelle ont été greffés par liaison de covalence des groupements sulfoisobutyl de telle sorte que le virus rabique se fixe sur ce support, et
b) on élue le virus de son support.

2. Un procédé selon la revendication 1, selon lequel le surnageant de culture des cellules infectées par le virus rabique est dépourvu de sérum animal ou de toute protéine sérique.

3. Un procédé selon la revendication 1 ou 2, selon lequel le surnageant de culture des cellules infectées par le virus rabique est dépourvu de toute protéine exogène d'origine animale.

4. Un procédé selon la revendication 3, selon lequel la concentration en protéines exogènes d'origine non animale dans le surnageant de culture de cellules infectées par le virus rabique est ≤ 15mg/l.

5. Un procédé selon l'une des revendications 1 à 4, selon lequel le surnageant de culture des cellules infectées par le virus rabique est dépourvu de tout produit exogène d'origine animale.

6. Un procédé selon l'une des revendications 1 à 5, selon lequel le surnageant de culture de cellules infectées par le virus rabique est préalablement clarifié avant d'être mis en contact avec le support de chromatographie échangeur de cations.

7. Un procédé selon l'une des revendications 1 à 6, **caractérisé en ce que** la quantité de virus mesurée dans l'éluât correspond à au moins 70 % de la quantité de virus mesurée dans le surnageant qui a été mis en contact avec le support chromatographique.

8. Un procédé selon la revendication 7, **caractérisé en ce que** la quantité de protéines totales mesurée dans l'éluât correspond à moins de 40 % de la quantité de protéines totales mesurée dans le surnageant qui a été mis en contact avec le support chromatographique et **en ce que** la quantité d'ADN mesurée dans l'éluât correspond à moins de 5 %, de préférence à moins de 2,5 % et de façon encore plus préférée à moins de 1 % de la quantité d'ADN mesurée dans le surnageant qui a été mis en contact avec le support chromatographique.

9. Un procédé selon l'une des revendications 1 à 8, selon lequel on concentre éventuellement l'éluât puis on le traite avec une nucléase.

10. Un procédé selon la revendication 9, selon lequel la nucléase est une endonuclease telle que la benzonase.

11. Un procédé selon l'une des revendications 1 à 10, selon lequel on soumet ensuite l'éluât à une ultra centrifugation sur un gradient de saccharose et on recueille la ou les fractions du gradient qui contiennent le virus purifié.

12. Un procédé selon l'une des revendications 1 à 11, selon lequel on inactive ensuite le virus rabique purifié au moyen d'un agent d'inactivation virale.

13. Un procédé selon la revendication 12, selon lequel l'agent d'inactivation virale est la β-propiolactone.

14. Un procédé selon l'une des revendications 1 à 13, selon lequel toutes les étapes du procédé sont réalisées au moyen de produits d'origine non animale.

15. Un procédé de fabrication d'un vaccin contre la rage selon lequel :
a) on infecte une culture de cellules avec le virus rabique,
b) on purifie le virus rabique à partir du surnageant de culture de cellules infectées selon un procédé tel que décrit dans l'une des revendications 1 à 14,
c) on mélange la suspension de virus purifié obtenue en b) dans un tampon de conservation, et
d) on répartit la suspension de virus purifié obtenue en c) sous la forme de vaccins uni-doses ou multi-doses.

16. Un procédé de fabrication d'un vaccin contre la rage selon lequel :
a) on infecte une culture de cellules avec le virus rabique,
b) on purifie le virus rabique à partir du surnageant de culture de cellules infectées selon un procédé tel que décrit dans l'une des revendications 1 à 14,
c) on mélange la suspension de virus purifié obtenue en b) dans un tampon de lyophilisation,
d) on répartit le mélange obtenu en c) sous la forme de vaccins unidoses ou multi-doses, et
e) on lyophilise les doses de vaccins.

## Patentansprüche

1. Verfahren zur Aufreinigung des Tollwutvirus, das einen einzigen Ionenaustauschchromatographieschritt umfasst, wobei es sich bei diesem Schritt um eine Kationenaustauschchromatographie handelt, bei der man:
a) den Kulturüberstand von mit diesem Virus infizierten Zellen mit einem Kationenaustauschchromatographieträger, der eine Polymethacrylatmatrix umfasst, auf die mittels kovalenter Bindung Sulfoisobutylgruppen so gepfropft sind, dass das Tollwutvirus auf diesem Träger immobilisiert wird, in Kontakt bringt und
b) das Virus von seinem Träger eluiert.

2. Verfahren nach Anspruch 1, bei dem der Kulturüberstand der mit dem Tollwutvirus infizierten Zellen frei von tierischem Serum oder jeglichem Serumprotein ist.

3. Verfahren nach Anspruch 1 oder 2, bei dem der Kulturüberstand der mit dem Tollwutvirus infizierten Zellen frei von jeglichem exogenen Protein tierischen Ursprungs ist.

4. Verfahren nach Anspruch 3, bei dem die Konzentration an exogenen Proteinen nicht tierischen Ursprungs im Kulturüberstand von mit dem Tollwutvirus infizierten Zellen ≤ 15mg/l beträgt.

5. Verfahren nach einem der Ansprüche 1 bis 4, bei dem der Kulturüberstand der mit dem Tollwutvirus infizierten Zellen frei von jeglichem exogenen Produkt tierischen Ursprungs ist.

6. Verfahren nach einem der Ansprüche 1 bis 5, bei dem der Kulturüberstand von mit dem Tollwutvirus infizierten Zellen zuerst geklärt wird, bevor er mit dem Kationenaustauschchromatographieträger in Kontakt gebracht wird.

7. Verfahren nach einem der Ansprüche 1 bis 6, **dadurch gekennzeichnet, dass** die in dem Eluat gemessene Virusmenge mindestens 70% der in dem Überstand, der mit dem Chromatographieträger in Kontakt gebracht worden ist, gemessenen Virusmenge entspricht.

8. Verfahren nach Anspruch 7, **dadurch gekennzeichnet, dass** die in dem Eluat gemessene Gesamtproteinmenge mindestens 40% der in dem Überstand, der mit dem Chromatographieträger in Kontakt gebracht worden ist, gemessenen Gesamtproteinmenge entspricht und dass die in dem Eluat gemessene DNA-Menge mindestens 5%, vorzugsweise mindestens 2,5%, noch stärker bevorzugt mindestens 1% der in dem Überstand, der mit dem Chromatographieträger in Kontakt gebracht worden ist, entspricht.

9. Verfahren nach einem der Ansprüche 1 bis 8, bei dem man das Eluat gegebenenfalls konzentriert und dann mit einer Nuklease behandelt.

10. Verfahren nach Anspruch 9, wobei es sich bei der Nuklease um eine Endonuklease wie Benzonase handelt.

11. Verfahren nach einem der Ansprüche 1 bis 10, bei dem man anschließend mit dem Eluat eine Ultrazentrifugation über einen Saccharosegradienten durchführt und die Fraktion(en) des Gradienten, die das aufgereinigte Virus enthält/enthalten, auffängt.

12. Verfahren nach einem der Ansprüche 1 bis 11, bei dem man das aufgereinigte Tollwutvirus anschließend mit einem Virusinaktivierungsmittel inaktiviert.

13. Verfahren nach Anspruch 12, wobei es sich bei dem Inaktivierungsmittel um β-Propiolacton handelt.

14. Verfahren nach einem der Ansprüche 1 bis 13, bei dem alle Schritte des Verfahrens mit Produkten nichttierischen Ursprungs durchgeführt werden.

15. Verfahren zur Herstellung eines Impfstoffs gegen Tollwut, bei dem man:
a) eine Zellkultur mit dem Tollwutvirus infiziert,
b) das Tollwutvirus aus einem Kulturüberstand von infizierten Zellen nach einem in einem der Ansprüche 1 bis 14 beschriebenen Verfahren aufreinigt,
c) die in b) erhaltene Suspension von aufgereinigten Viren mit einem Konservierungspuffer mischt, und
d) die in c) erhaltene Suspension des aufgereinigten Virus in Form von Einzeldosis- oder Mehrfachdosisimpfstoffen aufteilt.

16. Verfahren zur Herstellung eines Impfstoffs gegen Tollwut, bei dem man:
a) eine Zellkultur mit dem Tollwutvirus infiziert,
b) das Tollwutvirus aus einem Kulturüberstand von infizierten Zellen nach einem in einem der Ansprüche 1 bis 14 beschriebenen Verfahren aufreinigt,
c) die in b) erhaltene Suspension von aufgereinigten Viren mit einem Lyophilisierungspuffer mischt, und
d) die in c) erhaltene Mischung in Form von Einzeldosis- oder Mehrfachdosisimpfstoffen aufteilt, und
e) die Impfstoffdosen lyophilisiert.

## Claims

1. Method for purifying rabies virus, comprising a single ion-exchange chromatography step, said step being cation exchange chromatography according to which:
a) the supernatant of a culture of cells infected with this virus is brought into contact with a cation exchange chromatography support comprising a polymethacrylate matrix onto which sulfoisobutyl groups have been grafted by covalent bonding such that the rabies virus binds to this support, and
b) the virus is eluted from its support.

2. Method according to Claim 1, according to which the culture supernatant of the cells infected with the rabies virus is free of animal serum or of any serum protein.

3. Method according to Claim 1 or 2, according to which the culture supernatant of the cells infected with the rabies virus is free of any exogenous protein of animal origin.

4. Method according to Claim 3, according to which the concentration of exogenous proteins of non-animal origin in the culture supernatant of the cells infected with the rabies virus is ≤ 15 mg/l.

5. Method according to one of Claims 1 to 4, according to which the culture supernatant of the cells infected with the rabies virus is free of any exogenous product of animal origin.

6. Method according to one of Claims 1 to 5, according to which the culture supernatant of cells infected with the rabies virus is clarified before being brought into contact with the cation exchange chromatography support.

7. Method according to one of Claims 1 to 6, **characterized in that** the amount of virus measured in the eluate corresponds to at least 70% of the amount of virus measured in the supernatant that was brought into contact with the chromatographic support.

8. Method according to Claim 7, **characterized in that** the amount of total proteins measured in the eluate corresponds to less than 40% of the amount of total proteins measured in the supernatant that was brought into contact with the chromatographic support and **in that** the amount of DNA measured in the eluate corresponds to less than 5%, preferably to less than 2.5%, and even more preferably to less than 1% of the amount of DNA measured in the supernatant that was brought into contact with the chromatographic support.

9. Method according to one of Claims 1 to 8, according to which the eluate is optionally concentrated and then treated with a nuclease.

10. Method according to Claim 9, according to which the nuclease is an endonuclease such as benzonuclease.

11. Method according to one of Claims 1 to 10, according to which the eluate is then subjected to an ultracentrifugation on a sucrose gradient and the fraction(s) of the gradient which contain(s) the purified virus is (are) recovered.

12. Method according to one of Claims 1 to 11, according to which the purified rabies virus is then inactivated by means of a viral inactivation agent.

13. Method according to Claim 12, according to which the viral inactivation agent is β-propiolactone.

14. Method according to one of Claims 1 to 13, according to which all the method steps are carried out using products of non-animal origin.

15. Method for the manufacture of a rabies vaccine, according to which:
a) a culture of cells is infected with the rabies virus,
b) the rabies virus is purified from the infected-cell culture supernatant according to a method as described in one of Claims 1 to 14,
c) the suspension of purified virus obtained in b) is mixed into a storage buffer, and
d) the suspension of purified virus obtained in c) is divided up in the form of single-dose or multidose vaccines.

16. Method for the manufacture of a rabies vaccine, according to which:
a) a culture of cells is infected with the rabies virus,
b) the rabies virus is purified from the infected-cell culture supernatant according to a method as described in one of Claims 1 to 14,
c) the suspension of purified virus obtained in b) is mixed into a lyophilization buffer,
d) the mixture obtained in c) is divided up in the form of single-dose or multidose vaccines, and
e) the vaccine doses are lyophilized.
